# EUROPEAN PATENT APPLICATION

(11) **EP 1 870 712 A1**
(43) Date of publication of application: **26.12.2007**
(21) Application number: 06012594.5
(22) Date of filing: 20.06.2006
(51) Int. Cl.: G01N 33/86

(54) **Method for assaying a thrombotic event**

(71) Applicant: Universität Bern, 3012 Bern (CH)
(72) Inventor: Kohler, Hans-Peter, 3095 Spiegel bei Bern (CH); Schroeder, Verena, 3008 Bern (CH)

(57) **Abstract**

The invention provides a method of diagnosis for identifying a thrombotic event, in particular for identifying myocardial infarction, ischemic stroke, deep vein thrombosis, pulmonary embolism, or disseminated intravascular coagulation (DIC) in a human or animal, wherein a sample taken from the body of said human or animal is used to determine the coagulation factor XIII activation peptide (FXIII-AP) expressed differentially in body fluids and/or tissues of humans or animals suffering a thrombotic event.

Furthermore, the invention provides a kit of parts for identifying a thrombotic event in a human or animal, comprising specific detection means, in particular FXIII-AP binding agents for determining FXIII-AP levels in one or more of the above-mentioned samples.

In addition, the invention provides FXIII-AP binding agents, in particular polyclonal, monoclonal, hybrid, and synthetic antibodies specific for FXIII-AP.

## Description

### Field of the Invention

The present invention relates to a method of diagnosis for identifying a thrombotic event, in particular for identifying myocardial infarction, ischemic stroke, deep vein thrombosis, pulmonary embolism, or disseminated intravascular coagulation (DIC) in a human or animal, wherein a sample taken from the body of said human or animal is provided to determine the coagulation factor XIII activation peptide (FXIII-AP) expressed differentially in body fluids and/or tissues of humans or animals suffering a thrombotic event.

Furthermore, the present invention relates to a kit of parts for identifying a thrombotic event in a human or animal, comprising specific detection means, in particular FXIII-AP binding agents for determining FXIII-AP levels in one or more of the above-mentioned samples.

In addition, the present invention relates to FXIII-AP binding agents, in particular polyclonal, monoclonal, hybrid, and synthetic antibodies specific for FXIII-AP.

### Background of the Invention

Thrombotic diseases are the leading cause of morbidity and mortality in humans in the Western world. Arterial thrombotic diseases, such as coronary artery disease, cerebrovascular disease, or peripheral arterial vascular disease, develop over decades in consequence of atherosclerosis, and eventually culminate in acute events such as myocardial infarction or ischemic stroke. Venous thrombotic diseases become manifest in deep vein thrombosis and pulmonary embolism. Despite differences in the underlying pathophysiological mechanisms, both arterial and venous acute thrombotic events result from occlusion of a blood vessel by a hemostatic clot. Sepsis and systemic inflammation can lead to disseminated intravascular coagulation (DIC), a systemic activation of coagulation with formation of micro-thrombi at multiple sites in the body. Therefore, the hemostatic system is of particular importance in regard to prevention, pathophysiology, diagnosis, and therapy of thrombotic diseases. [1-4] (see list of references at the end of the description).

Hemostasis is based on coordinated interactions between vessel wall, platelets, plasmatic coagulation factors, and fibrinolysis. The coagulation cascade, which describes the sequential activation of plasmatic coagulation factors, represents the central part of this balanced system. Briefly, exposure of tissue factor due to damage of the vessel wall initiates the coagulation cascade. Formation and activation of different coagulation factor complexes, i.e. tenase complex and prothrombinase complex, result in formation of thrombin. Thrombin converts fibrinogen to fibrin and activates factor XIII (FXIII) which covalently cross-links soluble fibrin to a stable clot [5].

Thrombotic diseases are associated with activation of the coagulation cascade. Therefore, acute thrombotic events are diagnosed based on clinical symptoms and laboratory markers representing coagulation activation. Activation markers are prothrombin fragment 1+2 (F1+2), thrombin-antithrombin complex (TAT), and D-dimer, whereas D-dimer is the marker which is most commonly used in the clinic. F1+2 and TAT both represent activation of thrombin. Thrombin is a key enzyme of the coagulation cascade, and because of its different functions there exist several inhibitors for its neutralization. Therefore, by measuring thrombin activation, it is not known how much thrombin is really available for fibrin formation. D-dimer is a fibrin degradation product and is formed when fibrin is already degraded by fibrinolytic proteins. D-dimer indirectly reflects clot formation, however, it is a retrospective association, and formation of D-dimer depends on activity of the fibrinolytic system. Another problem of D-dimer testing is standardization since fibrin degradation leads to a mixture of different fibrin degradation fragments. These different fragments can possess different affinities to antibodies used in ELISA methods resulting in large variation depending on the assay used [6, 7].

An ideal marker of coagulation activation should occur late in the cascade and directly reflect clot formation, i.e. it should emerge at the time the clot is formed, and correlate with its extent.

FXIII is the final coagulation factor of the cascade. Its activation peptide (AP) may therefore be a candidate for such an ideal marker of coagulation activation. Activated factor XIII (FXIIIa), which is a transglutaminase, covalently cross-links fibrin polymers and incorporates antifibrinolytic proteins into fibrin. Only by these reactions the fibrin clot obtains sufficient stability and resistance to premature fibrinolysis. Accordingly, FXIII is a major player in any thrombo-embolic event, namely myocardial infarction, ischemic stroke, deep vein thrombosis, pulmonary embolism, and DIC.

FXIII zymogen circulates in plasma as a tetramer of two catalytic A-subunits and two carrier B-subunits (A₂B₂) [8, 9]. In plasma, thrombin initiates the physiological conversion of the FXIII zymogen into the active enzyme by cleavage of the N-terminal AP, resulting in FXIII A'₂B₂. This reaction is greatly enhanced by fibrin. Then, the dissociation of the A'- and B-subunits is induced by conformational changes due to the binding of Ca²⁺, again enhanced by fibrin. Finally, in the absence of the inhibitory B-subunits and in presence of Ca²⁺, the A'-subunits assume their active configuration (A*) [10].

Despite the increasing interest in FXIII, its numerous functions and its role in various diseases, several issues in particular regarding FXIII activation have not been resolved yet. It has been so far unknown, if in vivo the AP cleaved from plasmatic FXIII is actually released or remains associated with the activated protein. In the 1970s, Schwartz et al. [11] showed the electrophoretic patterns of non-activated and activated FXIII on polyacrylamide gels in sodium dodecyl sulfate. When activated by thrombin, the A-subunit decreases in molecular weight by 4000, indicating release of the AP. From the first three-dimensional structure of FXIII obtained from X-ray crystallography, Yee et al. [12] deduced that the AP blocks the access to the active site cavity, and therefore important conformational changes including removal or at least movement of the AP are required for FXIII activation. Later, however, X-ray crystallography of the FXIII A'₂ dimer after cleavage of the AP by thrombin gave some evidence that the AP is not released, since no large conformational changes in the protein were found, and the AP had almost the same conformation and occupied the same position as within the zymogen structure [13]. A revised model of FXIII activation suggests a role of γA/γ' fibrinogen in FXIII activation: After fibrin formation, the FXIII-AP is cleaved by thrombin but remains associated with the FXIII A'₂ subunits, and dissociation of the AP and the B-subunits is then accelerated by γA/γ' fibrinogen [14]. Some evidence for the release of FXIII-AP after its cleavage comes from in vitro experiments studying the kinetics of FXIII activation in a purified system, where free AP was detected by highpressure liquid chromatography (HPLC) [15]. Another study has described mass spectrometric detection of the AP after extensive processing of serum samples [16]. However, all experimental settings mentioned above involved special, non-physiological conditions, e.g. denaturating agents, high concentrations for crystallization, protein precipitation, chemical solvents, or high pressure. Therefore, the results may not apply to the in vivo situation. In summary, there exists no proof whether or not, under physiological conditions, the FXIII-AP is released into plasma upon FXIII activation.

To answer this question, specific antibodies against the FXIII-AP have been produced and a sensitive and specific ELISA method for direct detection of circulating FXIII-AP has been developed, and it has been found that the FXIII-AP is indeed released in vivo. This finding is believed to be of major clinical interest since it could serve as a new marker for acute thrombotic disease.

### Summary of the invention

In a first aspect, the present invention provides a method of diagnosis for identifying a thrombotic event in a human or animal which comprises determining in a sample taken from the body of said human or animal the concentration of coagulation factor XIII activation peptide (FXIII-AP).

Said thrombotic event includes,but is not limited to, myocardial infarction, ischemic stroke, deep vein thrombosis, pulmonary embolism and disseminated intravascular coagulation (DIC).

The aforesaid sample taken from the human or animal body which is used as substrate for evaluating the level of FXIII-AP is conveniently a sample of a tissue or of a body fluid, said body fluid including, but not being limited to, blood, blood plasma and blood serum.

The level of FXIII-AP is conveniently determined using an ELISA test based on FXIII-AP binding agents, said FXIII-AP binding agents including, but not being limited to, polyclonal, monoclonal, hybrid and synthetic antibodies specific for FXIII-AP.

In a further aspect, the present invention provides a test system for determining the level of FXIII-AP, said test system comprising
a) at least one coupled or uncoupled polyclonal, monoclonal, hybrid or synthetic antibody;
b) optionally a buffer system;
c) optionally a blocking agent; and
d) at least one substrate for the enzymes used in coupling to the antibodies or the analyte.

Said optional buffer can be, for example, PBS, TBS, or the like, and said optional blocking agent can be, for example, BSA, or the like.

The test system of the present invention can be in a form which can be applied in liquid, dried or lyophilized form.

A reagent containing analyte, also called flagged analyte, can conveniently be used in the test system of the present invention. Said reagent containing analyte can be FXIII-AP, or the sequence or part of the sequence of FXIII-AP, coupled with an enzyme, with particles (e.g. metal atoms, such as gold), with peptides, with electrochemically active substances, or with radioactive substances.

In the test system of the present invention for identifying a thrombotic event in a human or animal, the level of FXIII-AP can be modified in consequence of the event. Indeed the level of FXIII-AP can be increased and/or decreased.

The thrombotic event to be identified by the test system of the present invention includes, but is not limited to, myocardial infarction, ischemic stroke, deep vein thrombosis, pulmonary embolism, and disseminated intravascular coagulation (DIC).

In a still further aspect, the present invention provides monoclonal, polyclonal, hybrid and synthetic antibodies against the FXIII-AP variant SETSRTAFGGRRAVPPNNSNAAEDDLPTVELQGVVPR [SEQ.ID.NO:1] and monoclonal, polyclonal, hybrid and synthetic antibodies against the FXIII-AP variant
SETSRTAFGGRRAVPPNNSNAAEDDLPTVELQGLVPR [SEQ.ID.NO:2].

### Brief Description of the Figures

1 Structural analysis of the FXIII-AP.
   a) Structure of the FXIII A₂ homodimer deduced from its crystal structure. One monomer is represented in ribbons, the other is represented as a surface. The beginning and end of the AP are indicated by the black arrows (notice that the amino acids 1-4 and 37-38 are not displayed as they do not have coordinates attributed in the crystal structure).
   b) Putative conformation of the free AP with two β-sheets: a typical structure after 350 ns of molecular dynamics simulation.
2 Characterization of the FXIII-AP ELISA.
   a) Concentration of AP vs. OD. AP concentration ranged from 0.01-100 ng/ml in dilution buffer, FXIII-deficient plasma, and normal pool plasma. (Mean values from duplicates are shown.)
   b) Standard curve of the FXIII-AP ELISA. (Mean values from six replicates.)
3 Time course of FXIII-AP and FXIII A-subunit antigen levels after in vitro activation of citrated plasma.
   a) Without the fibrin polymerization inhibitor GPRP.
   b) With GPRP.
4 Immunological test systems.
   a) Sandwich ELISA.
   b) Competitive ELISA.
   c) Replacement ELISA.

### Detailed description of the Invention

### Structural analysis

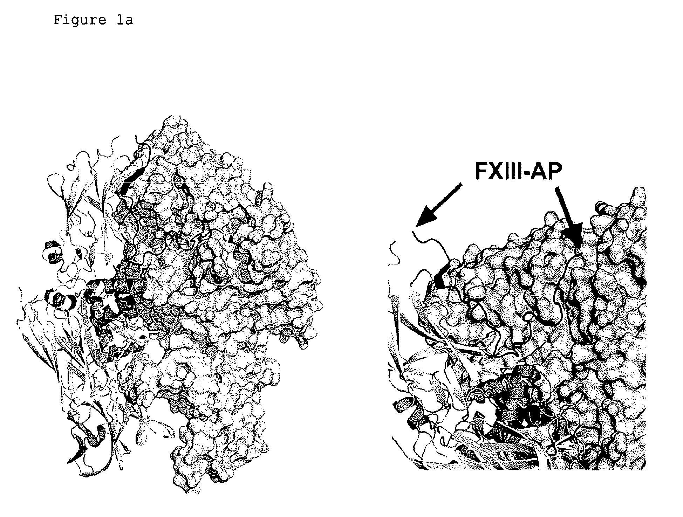

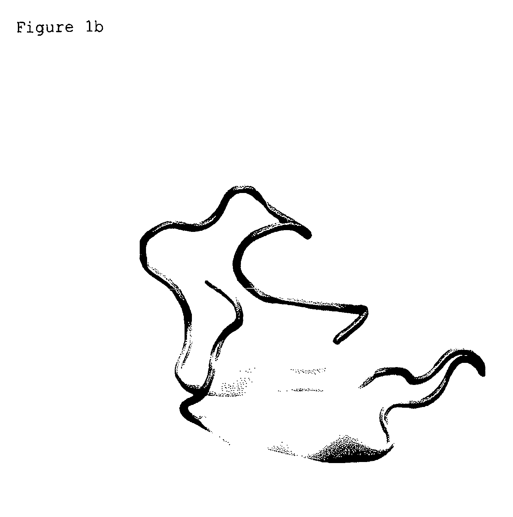

Figure 1a shows the conformation of the AP bound to the FXIII A₂ homodimer. In this condition, the amino acids 28-32 assume a β-sheet structure, but no further secondary structures are observed. After 175 ns of molecular dynamics (MD) simulation, the conformation of the free AP produced by an ab-initio modeling method reached a stable secondary structure with two β-sheets (Figure 1b). This secondary structure is conserved through the next 225 ns of MD-simulation. This structure may represent a putative conformation of the free AP and, as expected, the free peptide's structure is not similar to the structure of the AP within the FXIII A₂ homodimer. It is reasonable to conclude that generation of antibodies specific for the free AP might be feasible due to these putative differenced in conformation.

### ELISA development and characterization

Rabbits were successfully immunized with FXIII-AP, and polyclonal antibodies could be generated and purified. Mice were also successfully immunized with FXIII-AP, cells were fused, clones were grown and subcloned, and monoclonal antibodies could be harvested and purified.

An ELISA method, based on the sandwich principle, was then developed. For this first assay prototype, only polyclonal antibodies were used. Using two polyclonal antibodies from the same species, rabbit, one antibody was labeled with biotin for detection with the biotin/streptavidin system.

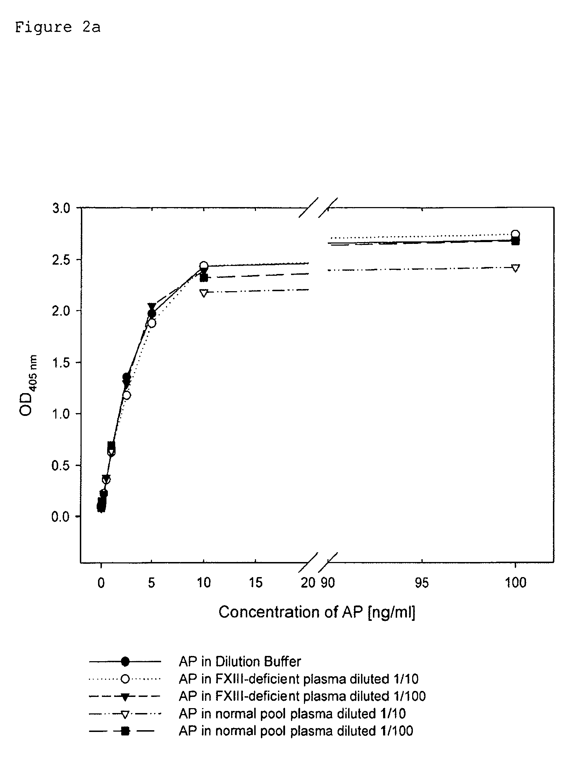

Figure 2a shows the AP-concentration vs. OD curves of the ELISA over a range of 0.01-100 ng/ml of AP diluted in dilution buffer, FXIII-deficient plasma, and normal pool plasma. The ELISA functions over a broad range and detects AP-concentrations as low as 0.01 ng/ml. Dilution of AP in FXIII-deficient plasma and normal pool plasma gave similar curves to AP diluted in buffer, which indicates that the immune reaction is independent of the presence of other plasma compounds including plasmatic FXIII. Blank values of the diluted plasmas were similar to the dilution buffer blank and gave no significant OD signals. It turned out possible to detect different AP-concentrations in FXIII-deficient plasma and normal pool plasma as accurately as in dilution buffer.

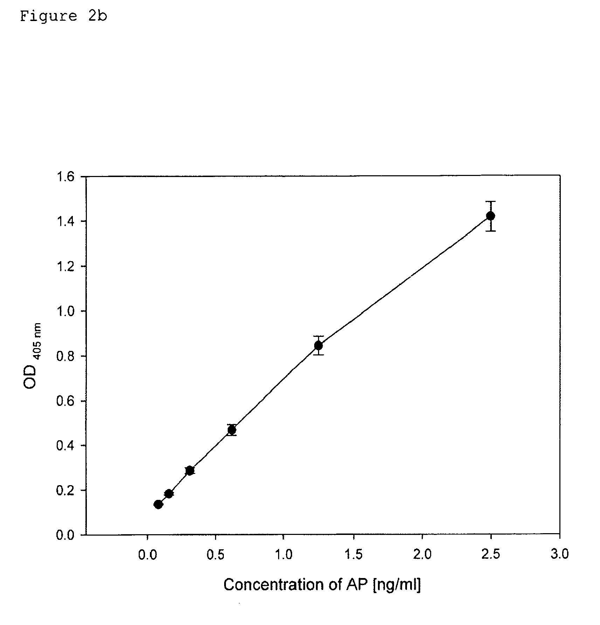

The detection limit of the ELISA was 0.01 ng/ml in dilution buffer and 0.1 ng/ml in plasma (diluted at least 1/10). Finally it turned out possible to define the standard curve of the ELISA, which is shown in Figure 2b, in the linear part of the concentration vs. OD curve. The AP-concentrations of the six standards were as follows: 2.5, 1.25, 0.625, 0.3125, 0.15625, and 0.078125 ng/ml. The linear correlation coefficient of the standard curve was 0.998.

### Determination of in vitro generated AP

In non-activated citrated plasma samples from healthy volunteers, normal FXIII A-subunit antigen levels were found (114%, 144%, and 147%, expressed as percentage of the International FXIII Standard), however no FXIII-AP could be detected. In the serum samples from the same individuals, there were measured high AP levels (86, 109, and 145 ng/ml) and low FXIII A-subunit antigen levels (2%, 4%, and <1%). In the serum samples kept for 4 hours at 4°C there were still 80% and in the samples kept at room temperature there were 65% of the original AP concentration.

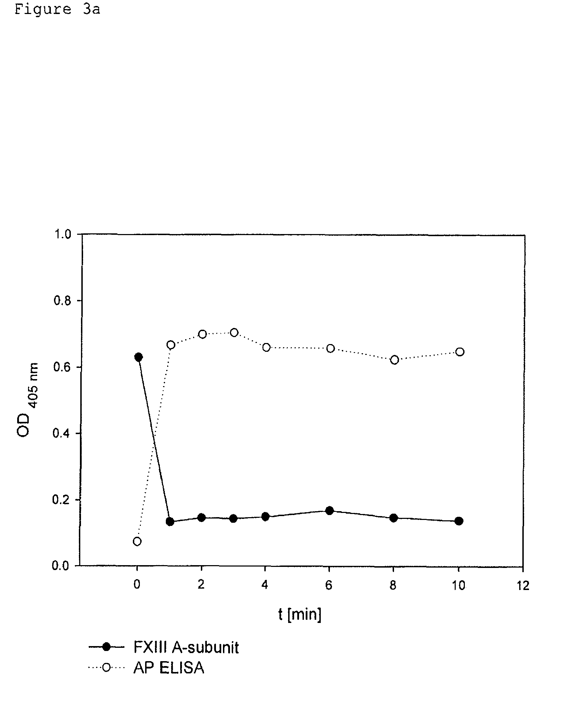

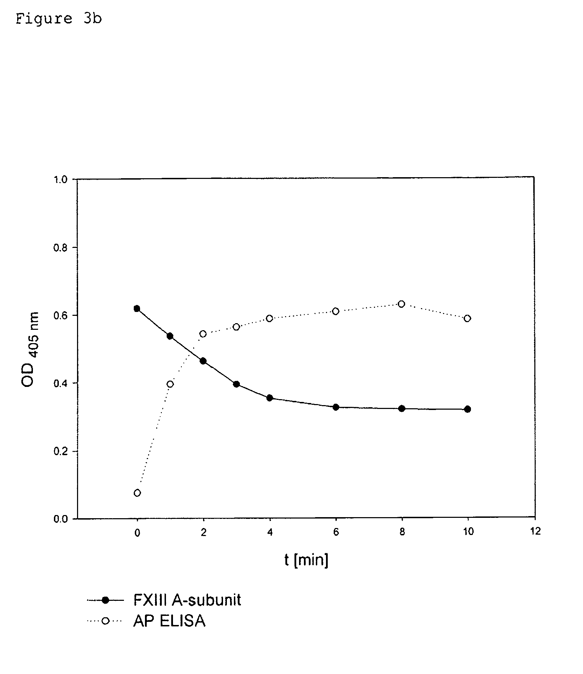

Figure 3 shows the time course of FXIII-AP and FXIII A-subunit antigen levels after in vitro activation of citrated plasma. Without addition of the fibrin polymerization inhibiting peptide, fast clot formation in the samples was observed. This is reflected by the ELISA results showing a fast increase in FXIII-AP and a fast decrease in FXIII A-subunit (Figure 3a). In samples without clot formation due to inhibition of fibrin polymerization, there were found similar AP levels, a less prominent decrease in FXIII A-subunit antigen levels, and the changes occurred slightly slower (Figure 3b).

### Determination of in vivo generated AP

It turned out possible to detect FXIII-AP directly in plasma samples from patients with acute myocardial infarction, without any prior in vitro activation of the samples. While no AP could be detected in plasma from healthy volunteers, AP levels of 2.5 and 20.4 ng/ml were found in patient samples. The presence of AP in the plasma samples from the patients indicates activation of coagulation in association with the myocardial infarction.

### Diagnosis of a thrombotic event

### Indication:

The diagnostic procedure is intended to be performed in a human or animal with a suspected thrombotic event such as myocardial infarction, ischemic stroke, deep vein thrombosis, pulmonary embolism, or disseminated intravascular coagulation (DIC).

### Diagnostic procedure:

A sample of body fluids and/or tissue from this human or animal is taken. In this sample, the FXIII-AP is determined using a test system as detailed below. In plasma from healthy individuals no AP was detectable. Therefore, any detectable AP in a plasma sample may reflect activation of the coagulation system and may support the diagnosis of an acute thrombotic event.

### Test system:

The test according to the test system of the present invention can be carried out either manually or with automated or semi-automated analyzers (e.g. coagulation analyzing systems, immunochemistry systems, ELISA-systems, etc.).

### Immunological test systems:

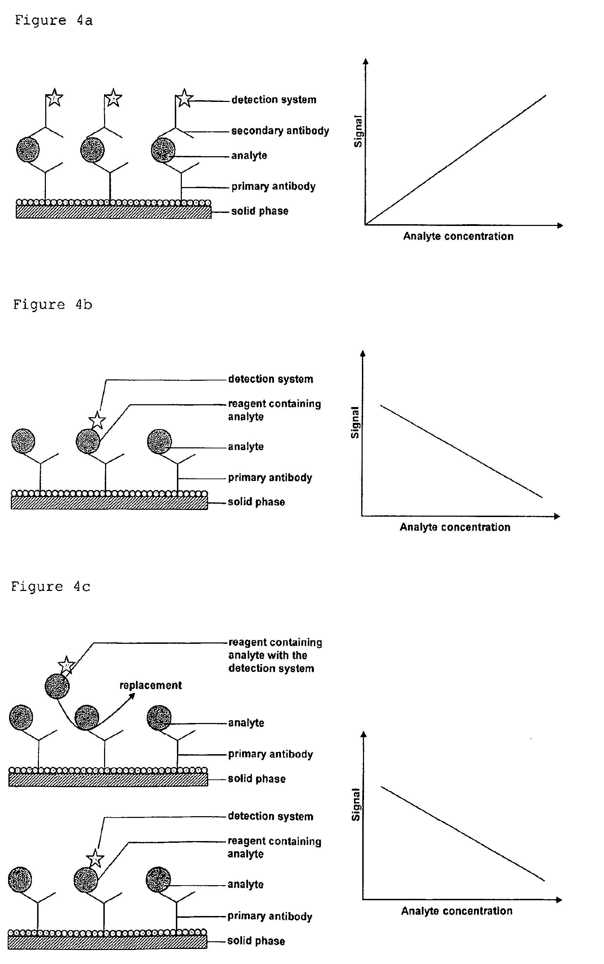

- Classical sandwich ELISA, brief description (Figure 4 a):
   - Coating of a primary antibody to a solid phase. The solid phase can be a microtiter plate, any kind of papers, particles etc. The antibodies can be polyclonal or monoclonal, complete antibodies or fragments of antibodies, hybrid antibodies, synthetic antibody analoga, etc.
   - Incubation with the body fluid or tissue that potentially contains unknown concentrations of the analyte, in this case the FXIII-AP. The fluid can be any kind of body fluid, such as blood, plasma, serum, spinal fluid, etc.
   - Detection of bound analyte with a second antibody. The antibodies can be polyclonal or monoclonal, complete antibodies or fragments of antibodies, hybrid antibodies, synthetic antibody analogs, etc. The antibodies can be coupled with substances for detection of analyte bound antibodies. Those substances can be enzymes, metal atoms, peptides, electrochemically active substances, etc.
   - Use of an appropriate detection system of bound secondary antibodies.
   - The test system results in a direct proportional correlation of analyte concentration and signal.
- Competitive ELISA-systems, brief description (Figure 4 b):
   - Coating of antibodies to a solid phase. The solid phase can be a microtiter plate, any kind of papers, particles etc. The antibodies can be polyclonal or monoclonal, complete antibodies or fragments of antibodies, hybrid antibodies, synthetic antibody analogs, etc.
   - Incubation with the body fluid or tissue that potentially contains unknown concentrations of the analyte, in this case the FXIII-AP, in combination with a reagent that contains the analyte, mostly a fixed concentration of the analyte. The fluid can be any kind of body fluid, such as blood, plasma, serum, spinal fluid, etc. The reagent can contain a synthetic form of the analyte, any kind of analyte analogs or analyte of natural origin. The analyte used for the reagent can be coupled with substances for detection of bound analyte. Those substances can be enzymes, metal atoms, peptides, electrochemically active substances, radioactive substances, etc.
   - Use of an appropriate detection system of bound analyte.
   - The test system results in a indirect proportional correlation of analyte concentration and signal.
- Replacement ELISA-systems, brief description (Figure 4 c):
   - Coating of antibodies to a solid phase. The solid phase can be a microtiter plate, any kind of papers, particles etc. The antibodies can be polyclonal or monoclonal, complete antibodies or fragments of antibodies, hybrid antibodies, synthetic antibody analogs, etc.
   - Incubation with the body fluid or tissue that potentially contains unknown concentrations of the analyte, in this case the FXIII-AP. The fluid can be any kind of body fluid, such as blood, plasma, serum, spinal fluid, etc.
   - Incubation of a reagent containig analyte, in this case the FXIII-AP, mostly in a fixed concentration. The reagent can contain a synthetic form of the analyte, any kind of analyte analogs or analyte of natural origin. The analyte used for the reagent can be coupled with substances for detection of bound analyte. Those substances can be enzymes, metal atoms, peptides, electrochemically active substances, radioactive substances, etc.
   - Either direct detection of bound reagent analyte to the primary antibody or detection of bound analyte with a second antibody. The antibodies can be polyclonal or monoclonal, complete antibodies or fragments of antibodies, hybrid antibodies, synthetic antibody analogs, etc. The antibodies can be coupled with substances for detection of analyte bound antibodies. Those substances can be enzymes, metal atoms, peptides, electrochemically active substances, etc.
   - Use of an appropriate detection system of bound secondary antibodies.
   - The test system results in an indirect proportional correlation of analyte concentration and signal.
- Immune detection systems can be carried out without a solid phase. They can be carried out in a total liquid system.
- Immune detection systems can be carried out with dry chemistry systems, like paper strip systems, cartridge systems etc.

It has been shown for the first time that upon activation by thrombin FXIII-AP is released into plasma where it can be directly quantified by a sensitive and specific ELISA method.

The conformation of the AP bound to FXIII has been compared with the putative conformation of the free AP by using an ab-initio modelling method. As expected conformational differences were observed between the free AP showing two β-sheet structures and the bound AP showing only β-sheet but no further secondary structures. Despite the limitations of ab initio modelling methods, these structural considerations allowed the assumption that the conformations of the two FXIII-AP forms are different in vivo. The conclusion was reached that it may be feasible to raise antibodies which are specific for the free FXIII-AP, provided that animals develop an immune response to the AP. Indeed, there were obtained polyclonal antibodies against FXIII-AP that do not cross-react with the non-activated FXIII molecule, and this made it possible to develop the first sensitive and specific immunological assay for FXIII-AP detection.

The questions on the AP's fate after FXIII activation in plasma can now be answered: Both after plasma FXIII activation by thrombin and after contact activation of the coagulation cascade to gain serum, high FXIII-AP levels can be measured in plasma/serum. At the same time, only traces of the FXIII A-subunit antigen can be detected after activation. This is consistent with the observations that activated FXIII tightly binds to fibrin, becomes incorporated into the clot and is removed from circulation [8, 9]. When clot formation was prevented by addition of GPRP peptide, almost the same levels of AP were found, but there was still some FXIII A-subunit antigen detectable. These findings demonstrate that the FXIII-AP is released into plasma/serum and does not remain attached to its parent protein. If that was the case, one could expect to find only traces of AP in clotted samples, with the AP sticking to activated FXIII trapped within the fibrin network, but higher AP levels in the non-clotted GPRP samples.

Furthermore, the detection of FXIII-AP in non-activated plasma samples from patients with a putative thrombotic event indicates that in vivo generated, free AP circulates in human plasma and can be detected with the above ELISA. Although additional studies may be required to further confirm this preliminary finding, there are good reasons for hypothesizing that FXIII-AP represents a novel marker in acute thrombotic events. This marker is believed to be of major clinical importance, since it would represent the final activation step of the coagulation cascade, and it would be the first marker which is generated exactly at the time of clot formation.

Being reasonably stable over several hours, the FXIII-AP fulfills one of the prerequisites of a diagnostic marker and should therefore be measurable in fresh plasma samples for diagnosis of acute thrombotic events.

### Examples

### 1. Structural analysis

Structural analysis was based on the crystal structure of the FXIII A₂-homodimer obtained from the Protein Data Bank PDB (PDB Entry 1F13). The amino acid sequence of the activation peptide (amino acids 1-37) was subjected to an ab-initio molecular modeling method. The model was then optimized by molecular dynamics simulations.

### 2. Antibody production

Monoclonal and polyclonal antibodies against FXIII-AP were developed. In the FXIII gene there is a mutation leading to an amino acid exchange of Valine to Leucine at position 34 within the FXIII-AP. Since this mutation is common in the normal population (approx. 50% carry this mutation), antibodies against both FXIII-AP variants [17, 18] were raised. The FXIII-AP was synthesized according to the published sequence (SETSRTAFGGRRAVPPNNSNAAEDDLPTVELQGVVPR, PDB Entry 1F13 [SEQ.ID.NO:1]), and with the mutant amino acid for the mutant FXIII-AP variant (sequence SETSRTAFGGRRAVPPNNSNAAEDDLPTVELQGLVPR [SEQ.ID.NO:1]). After HPLC purification, the APs were conjugated to keyhole limpet hemocyanin (KLH) as carrier protein.

To obtain polyclonal antibodies, New Zealand White rabbits were repeatedly immunized (either with the individual AP variants, or co-immunized with a mixture of both AP variants), and several bleeds were taken between Week 5 and 10 after the first immunization. Antibody titers were determined by ELISA against the AP. The pooled bleeds were protein G affinity purified and affinity purified against the AP.

An aliquot of one polyclonal antibody was labeled with biotin using the EZ-Link^{®} Sulfo-NHS-LC-Biotinylation Kit (Pierce, Rockford, USA).

To obtain monoclonal antibodies against FXIII-AP, Balb/c mice were repeatedly co-immunized with a mixture of both AP variants. After six weeks, the spleen cells from the mouse with the highest antibody titer were isolated and fused with myeloma cells. Clones were grown and tested against FXIII-AP and the whole FXIII protein. Clones that were positive against FXIII-AP and negative against FXIII protein were subcloned twice. Clones with the highest reactivity against FXIII-AP and the lowest reactivity against FXIII protein were selected for large-scale production with subsequent protein G affinity purification.

### 3. ELISA development

A sandwich-type ELISA was employed using the above polyclonal rabbit anti-FXIII-AP antibodies [or monoclonal antibodies], one of them biotinylated. The following assay procedure was applied:
Ninety-six well microtiter plates (Maxisorp, Nunc, Roskilde, Denmark) were coated with 100 µl per well of the first polyclonal rabbit anti-FXIII-AP antibody (concentration 1 µg/ml in coating buffer containing 50 mM sodium carbonate, pH 9.6). The plate was incubated at room temperature on a shaker for 1 h, followed by overnight incubation at 4 °C. After five washing steps with 200 µl of washing buffer (50 mM Tris-HCl, 150 mM NaCl, 0.1% Tween-20, 0.01% sodium azide, pH 7.5), the plate was blocked with 200 µl of blocking buffer (50 mM Tris-HCl, 150 mM NaCl, 1% bovine serum albumine, 0.01% sodium azide, pH 7.5) and it was incubated at room temperature on a shaker for 1 h. After five washing steps, standards and plasma or serum samples were diluted appropriately with dilution buffer (50 mM Tris-HCl, 150 mM NaCl, 0.1% bovine serum albumine, 0.01% sodium azide, pH 7.5) and 100 µl was loaded in duplicate onto the plate. After 1 h of incubation at room temperature on a shaker, the plate was washed five times, 100 µl of the biotinylated polyclonal rabbit anti-FXIII-AP antibody (1 µg/ml in dilution buffer) was loaded onto the plate, and it was incubated again for 1 h. After five washing steps, 100 µl of streptavidine-alkaline-phosphatase conjugate (10 µg/ml in dilution buffer; Sigma, Fluka Chemie GmbH, Buchs, Switzerland) were loaded onto the plate and it was incubated again for 1 h. After five washing steps, the plate was developed with 100 µl of p-nitrophenyl phosphate (1 mg/ml in 1 M diethanolamine containing 0.5 mM MgCl₂, pH 9.8; Sigma) and it was incubated at room temperature on a shaker. The color-developing reaction was stopped after exactly 10 minutes with 100 µl of 4 M NaOH, and optical density (OD) values were measured on a microplate reader at 405 nm with a reference filter at 550 nm (Tecan SLT Rainbow Reader with Magellan Software V1.11, Tecan Austria GmbH, Grödig, Austria).

Specificity and sensitivity of the above ELISA method were assessed by measuring FXIII-AP dilutions in dilution buffer, FXIII deficient plasma, and normal pool plasma. The synthetic FXIII-AP was diluted to final concentrations ranging from 1 µg/ml to 10 pg/ml in dilution buffer, FXIII deficient plasma (which was diluted 1/10 and 1/100), and in normal pool plasma (diluted 1/10 and 1/100). In each dilution series, a blank was included without FXIII-AP. From the resulting concentration vs. OD curves, linear range and detection limit of the ELISA were deduced, and the standard curve was defined.

### 4. Determination of in vitro generated AP

Blood from healthy volunteers was taken into 5 ml-tubes (Monovette^{®}, Sarstedt AG, Sevelen, Switzerland) containing 0.5 ml trisodium citrate solution (0.106 mol/l) for plasma separation or clot activator beads for serum separation. The plasma tubes were centrifugated immediately at 2000 g for 20 minutes and the citrated plasma was then separated. Serum tubes were allowed to stand for 30 minutes before centrifugation (2000 g, 20 minutes) to let coagulation proceed.

After centrifugation of the serum samples, one alilquot of each serum sample was immediately analyzed, two other aliquots were kept for 4 hours at 4°C in the fridge and at room temperature.

For in vitro generation of AP, citrated plasma was activated with thrombin (Sigma), and the reaction was stopped after different time points with the thrombin inhibitor Pro-Phe-Arg-chloromethylketone (PPACK; Bachem, Bubendorf, Switzerland). The experiment was performed in two parallel series, once with and once without the fibrin polymerization inhibiting peptide Gly-Pro-Arg-Pro-amide acetate (GPRP; Bachem [SEQ.ID.N0:3]). 100 µl of citrated plasma were mixed with 50 µl of GPRP (5 mg/ml in tris-buffered saline, TBS) or with 50 µl of TBS for the series without GPRP. Activation was effected with 50 µl of thrombin (10 U/ml in TBS) or by addition of 50 µl of TBS for the non-activated control. The reactions were stopped after 1, 2, 3, 4, 6, 8, and 10 minutes by addition of 50 µl of PPACK (140 µg/ml). The clotted samples without GPRP were centrifugated at 2000 g for 10 minutes and the supernatant was separated. For the AP ELISA, the activated plasma samples were diluted in dilution buffer to a final dilution of 1/50. Serum was directly diluted 1/50 in dilution buffer.

In all samples, FXIII A-subunit antigen levels were also determined by an in-house ELISA [19].

### 5. Determination of in vivo generated AP

FXIII-AP was measured in plasma from two patients admitted to the hospital because of acute myocardial infarction. Citrated plasma samples, which had been stored frozen at -80 °C from a previous study, were thawed and diluted 1/10 in dilution buffer for the AP ELISA. Plasma from healthy volunteers served as control.

### References

1 Libby P. Current concepts of the pathogenesis of the acute coronary syndromes. Circulation 2001; 104: 365-72.
2 Rauch U, Osende JI, Fuster V, Badimon JJ, Fayad Z, Chesebro JH. Thrombus formation on atherosclerotic plaques: pathogenesis and clinical consequences. Ann Intern Med 2001; 134: 224-38.
3 Rosendaal FR. Venous thrombosis: a multicausal disease. Lancet 1999; 353: 1167-73.
4 Goldhaber SZ. Pulmonary embolism. N Engl J Med 1998; 339: 93-104.
5 Dahlbäck B. Blood coagulation. Lancet 2000; 355: 1627-32.
6 Bauer KA. Activation markers of coagulation. Baillieres Best Pract Res Clin Haematol 1999; 12: 387-406.
7 Lowe GD. Fibrin D-dimer and cardiovascular risk. Semin Vasc Med 2005; 5: 387-98.
8 Ichinose A. The physiology and biochemistry of factor XIII. In: Haemostasis and Thrombosis. Bloom AL, Forbes CD, Thomas DP, Tuddenham EGD, eds. 3rd edn, Vol. 1. Edinburgh: Livingstone. 1994; 531-46.
9 Muszbek L, Ádány R, Mikkola H. Novel aspects of blood coagulation factor XIII. I. Structure, distribution, activation, and function. Crit Rev Clin Lab Sci 1996; 33: 357-421.
10 Lorand L. Factor XIII: Structure, activation, and interactions with fibrinogen and fibrin. Ann N Y Acad Sci 2001; 936: 291-311.
11 Schwartz ML, Pizzo SV, Hill RL, McKee PA. The subunit structures of human plasma and platelet factor XIII (fibrin-stabilizing factor). J Biol Chem 1971; 246: 5851-4.
12 Yee VC, Pedersen LC, Le Trong I, Bishop PD, Stenkamp RE, Teller DC. Three-dimensional structure of a transglutaminase: Human blood coagulation factor XIII. Proc Natl Acad Sci USA 1994; 91: 7296-7300.
13 Yee VC, Pedersen LC, Bishop PD, Stenkamp RE, Teller DC. Structural evidence that the activation peptide is not released upon thrombin cleavage of factor XIII. Thromb Res 1995; 78: 389-97.
14 Moaddel M, Falls LA, Farrell DH. The role of γA/γ' fibrinogen in plasma factor XIII activation. J Biol Chem 2000; 275: 32135-40.
15 Ariëns RAS, Philippou H, Chandrasekaran N, Weisel JW, Lane DA, Grant PJ. The factor XIII V34L polymorphism accelerates thrombin activation of factor XIII and affects cross-linked fibrin structure. Blood 2000; 96:988-95.
16 Tammen H, Möhring T, Kellmann M, Pich A, Kreipe HH, Hess R. Mass spectrometric phenotyping of Val34Leu polymorphism of blood coagulation factor XIII by differential peptide display. Clin Chem 2004; 50: 545-51.
17 Mikkola H, Syrjälä M, Rasi V, Vahtera E, Hämäläinen E, Peltonen L, Palotie A. Deficiency in the A-subunit of coagulation factor XIII: two novel point mutations demonstrate different effects on transcript levels. Blood 1994; 84: 517-25.
18 Attié-Castro FA, Zago MA, Lavinha J, Elion J, Rodriguez-Delfin L, Guerreiro JF, Franco RF. Ethnic heterogeneity of the factor XIII Val34Leu polymorphism. Thromb Haemost 2000; 84: 601-3.
19 Ariëns RAS, Kohler HP, Mansfield MW, Grant PJ. Subunit antigen and activity levels of blood coagulation factor XIII in healthy individuals. Relation to sex, age, smoking, and hypertension. Arterioscler Thromb Vasc Biol 1999; 19: 2012-6.

## Claims

1. A method of diagnosis for identifying a thrombotic event in a human or animal which comprises determining in a sample taken from the body of said human or animal the concentration of coagulation factor XIII activation peptide (FXIII-AP).

2. A method according to claim 1 wherein said thrombotic event is myocardial infarction, ischemic stroke, deep vein thrombosis, pulmonary embolism or disseminated intravascular coagulation (DIC).

3. A method according to claim 1 or claim 2 wherein a sample of a tissue or of a body fluid is used as substrate for evaluating the level of FXIII-AP.

4. A method according to claim 3 wherein said body fluid is blood, blood plasma or blood serum.

5. A method according to any one of claims 1 to 4 wherein the level of FXIII-AP is determined using an ELISA test based on FXIII-AP binding agents.

6. A method according to claim 5 wherein said FXIII-AP binding agents are polyclonal, monoclonal, hybrid or synthetic antibodies specific for FXIII-AP.

7. A test system for determining the level of FXIII-AP comprising
a) at least one coupled or uncoupled polyclonal, monoclonal, hybrid or synthetic antibody;
b) optionally a buffer system;
c) optionally a blocking agent; and
d) at least one substrate for the enzymes used in coupling to the antibodies or the analyte.

8. A test system according to claim 7 wherein said optional buffer is PBS or TBS.

9. A test system according to claim 7 or claim 8 wherein said optional blocking agent is BSA.

10. A test system according to any one of claims 7 to 9 which is in a form which can be applied in liquid, dried or lyophilized form.

11. A test system according to any one of claims 7 to 10 wherein a reagent containing analyte is used.

12. A test system according to claim 11 wherein said reagent containing analyte is the whole sequence of FXIII-AP or part of the sequence of FXIII-AP, coupled with an enzyme, with peptides, with particles, with electro-chemically active substances, or with radioactive substances.

13. A test system according to claim 12 wherein said particles consist of gold.

14. A test system according to any one one of claims 7 to 13 for identifying a thrombotic event in a human or animal wherein, in consequence of the event, the level of FXIII-AP can be modified.

15. A test system according to claim 14 wherein the level of FXIII-AP can be increased and/or decreased.

16. A test system according to claim 14 or claim 15 wherein the thrombotic event is myocardial infarction, ischemic stroke, deep vein thrombosis, pulmonary embolism, or disseminated intravascular coagulation (DIC).

17. Monoclonal, polyclonal, hybrid and synthetic antibodies against the FXIII-AP variant
SETSRTAFGGRRAVPPNNSNAAEDDLPTVELQGVVPR [SEQ.ID.NO:1].

18. Monoclonal, polyclonal, hybrid and synthetic antibodies against the FXIII-AP variant
SETSRTAFGGRRAVPPNNSNAAEDDLPTVELQGLVPR [SEQ.ID.NO:2].


SEQUENCE LISTING

<110> Universität Bern

<120> Method for assaying a thrombotic event

<130> P15514EP00

<140> EP 06012594.5
<141> 2006-06-06

<160> 3

<170> PatentIn Ver. 2.1

<210> 1
<211> 37
<212> PRT
<213> Homo sapiens

<400> 1
Ser Glu Thr Ser Arg Thr Ala Phe Gly Gly Arg Arg Ala Val Pro Pro
  1               5                  10                  15

Asn Asn Ser Asn Ala Ala Glu Asp Asp Leu Pro Thr Val Glu Leu Gln
             20                  25                  30

Gly Val Val Pro Arg
         35

<210> 2
<211> 37
<212> PRT
<213> Homo sapiens

<400> 2
Ser Glu Thr Ser Arg Thr Ala Phe Gly Gly Arg Arg Ala Val Pro Pro
  1               5                  10                  15

Asn Asn Ser Asn Ala Ala Glu Asp Asp Leu Pro Thr Val Glu Leu Gln
             20                  25                  30

Gly Leu Val Pro Arg
         35

<210> 3
<211> 4
<212> PRT
<213> Homo sapiens

<220>
<221> MOD_RES
<222> (4)
<223> AMIDATION

<400> 3
Gly Pro Arg Pro
1